# EUROPEAN PATENT APPLICATION

(11) **EP 1 566 169 A1**
(43) Date of publication of application: **24.08.2005**
(21) Application number: 05002571.7
(22) Date of filing: 08.02.2005
(51) Int. Cl.: A61K 7/48

(54) **Cosmetic compositions containing combinations of Bupleurum species extracts and Salvia miltiorrhiza extracts for the treatment and reduction of blemishes caused by cellulite**

(30) Priority: 10.02.2004 IT MI20040214
(71) Applicant: ABOCA S.p.A., 52037 San Sepolcro (Ar) (IT)
(72) Inventor: Mercati, Valentino, 52037 San Sepolcro (AR) (IT)
(74) Representative: Minoja, Fabrizio

(57) **Abstract**

Cosmetic compositions for the treatment and reduction of blemishes caused by cellulite containing combinations of *Bupleurum* species and *Salvia milthiorriza*. Said combinations are particularly advantageous, in that allow to obtain remarkably higher effects than those observed following administration separated of each single component vegetable.

## Description

### Field of invention

The present invention relates to cosmetic compositions for the treatment of the rippled, dimpled appearance caused by cellulite.

### Background of the invention

Cellulite is a progressive alteration of the subcutaneous panniculus adiposus which involves an initial degeneration of the ground substance of the connective tissue, followed by degeneration of the collagen fibres and fragmentation of the elastic fibres, with consequent cicatricial sclerosis. The process is manifested schematically in three stages, known as oedematous, fibrous and cicatricial.

At the first stage water retention occurs, with consequent congestion and adverse effects on metabolic turnover. At the second stage the connective tissue undergoes a progressive alteration, with degeneration and disappearance of the collagen fibres, fragmentation of the elastic fibres, hypertrophy of the adipose cells and the formation of micro- and macro-nodules. At the third stage the connective tissue undergoes fibre-sclerotic degeneration, compressing all the structures present in the dermis and hypodermis and adversely affecting the microcirculation and trophism of the tissues.

Cellulite has a number of causes, associated with alterations in the metabolism, non correct diet and lack of exercise, and with physiological phenomena relating to the hormones and the subcutaneous structure typical of the female sex. A correct diet and regular exercise can therefore help to reduce or limit this phenomenon, at least to some extent. Moreover, an adjuvant action can be performed by natural substances, usually of plant origin, which aid lipolysis and improve the characteristic "orange-peel" appearance of skin suffering from cellulite. Numerous anti-cellulite cosmetic products on the market are based on natural substances, especially plant extracts, which mainly perform an action that improves the microcirculation.

A good anti-cellulite cosmetic should perform the following functions:
a) improve the microcirculation of the surface capillaries;
b) improve the typical "orange-peel" appearance of skin with cellulite;
c) reduce the external visibility and tactile perception of the fat nodules encapsulated in the subcutaneous fibrous tissue (connective tissue) which are typical of this state of skin imbalance;
d) promote the normal lipid metabolism, ie. hydrolysis of the excess triglycerides accumulated in the superficial adipocytes.

Unfortunately, few plant extracts are able to perform these functions without causing toxic or side effects.

Cosmetic compositions based on extracts of *Bupleurum spp* or sage extracts are known for the treatment of blemishes caused by cellulite.

*Bupleurum falcatum (fam. Apiaceae* or *Umbelliferae)* is a plant reported in the Chinese pharmacopoeia. The species described are *Bupleurum chinense DC* (*B. falcatum auct. Sin. Non L.*), and *Bupleurum scorzonerifolium Willd.* (*B. faleatum var. scorzonerifolium* (willd.) Ledeb.), which differ in terms of morphology, origin and form of the roots, which are the part of the plant from which the active ingredients can be extracted, In practice, both species are considered equivalent to *B. falcatum L. var. scorzonerifolium.*

The main active constituents of the roots of *Bupleurum spp* are triterpene saponins (Yamamoto et al., *Arzneim.-Forsch.* 25 (7):1021-3, 1975), also called saikosides or saikogenins; the plant contains between 1.5% and 8% of these substances, The anti-inflammatory activity of the extracts has been demonstrated by studies in vivo (Bermejo Benito P, et al. *Life Sci.* 63(13): 1147-56, 1998). The anti-inflammatory power of the saikosaponins is similar to that of prednisolone. The proven efficacy of saikosaponin A as an antagonist of the action of histamine and the allergic mediators also explains its anti-inflammatory activity.

It has been suggested that its anti-inflammatory effects are due to inhibition of the arachidonic acid metabolism cascade. Moreover, the saikosaponins help to maintain vascular homeostasis by inhibiting ADP-induced platelet activation, with a dose-dependent effect and to an extent comparable to that of aspirin.

The saikosaponins (especially A, B1 and D, and to a lesser extent B2 and C) have proved to be powerful stimulators of PGE₂ release. Other active ingredients extracted from the root, such as bupleuran 2IIc, stimulate the anti-inflammatory response mediated by the lymphocytes, Finally, a recent study demonstrates the inhibiting effect of *Bupleurum* saponins on cell adhesion, another marker for inflammation (Ahn BZ, et al. *Planta Med.* 64(3):220-4, 1998).

*Salvia milthiorriza Bunge* (Chinese red sage), a plant belonging to the Labiatae family, is found almost exclusively in China. The active part of the plant is the roots, which contain dicarbonyl quinone compounds (diterpene quinones) called tanshinones; these may be substituted by two alcohol groups, in which case they are called tanshindiols. The main components, called tanshinone I, tanshinone II, cryptotanshinone, etc., were described for the first time by Japanese researchers, and some 40 different structures have been discovered to date. The total tanshinone content is approx. 1%. The extracts also contain diterpenes (ferruginol), phenols (salvianolic acid) and other quinones (miltirone). Their activity seems to be associated with the molecules with quinone groups. *Salvia milthiorriza* was used in traditional Chinese medicine to treat blood diseases, coronary disorders, hepatitis, haemorrhage, menstrual disorders and oedema. The extracts also inhibit platelet aggregation, which suggests their use in coronary disease. The tanshinones isolated have also proved able to dilate the blood vessels and promote diuresis. Sage extracts are also effective in the treatment of microcirculatory disorders (Microcirculation Research Group, Dept of Pathophysiology, Shangai First Medical College, Shanghai, *Chinese Medical Journal* 4(3):191-5, 1978).

EP 0 692 250 B1 discloses pharmaceutical and/or cosmetic formulations for the topical use containing esculoside in combination with adenylate cyclase stimulators and/or phosphodiesterase inhibitors and/or lipolytic agents as active ingredients. The preferred adenylate cyclase stimulators are forskolin and diterpenes of *Salvia milthiorriza,* used either individually or in combination.

Although combinations of extracts of *Bupleurum falcatum* and *Salvia milthiorriza* are described in the literature as aiding the bladder and kidney functions and alleviating pain, their combination is not described in cosmetic formulations designed to reduce, and limit blemishes caused by cellulite.

### DETAILED DESCRIPTION OF THE INVENTION

It has now been found that combinations of *Bupleurum falcatum* and *Salvia milthiorriza* extracts can be advantageously used to prepare cosmetic compositions designed to treat blemishes caused by cellulite.

This invention therefore relates to anti-cellulite cosmetic compositions containing combinations of *Bupleurum falcatum* and *Salvia milthiorriza* extracts.

As used herein, the terms *"Bupleurum falcatum"* and *"Salvia milthiorriza"* extracts mean lyophilized extracts or other extraction products, or extracts titrated in active ingredients (saikosaponins in the case of *Bupleurum spp* and diterpene quinones in the case of *Salvia milthiorriza*).

The total content of *Bupleurum, falcatum* and *Salvia milthiorriza* extracts in the compositions according to the invention is between 0.1% and 30% by weight, and preferably between 0.1 and 10% by weight. In particular, the titrated extracts, preferably lyophilized extracts, of *Bupleurum spp* and *Salvia milthiorriza* are each contained in the compositions according to the invention at a concentration of between 0.01 and 10%, and preferably between 0.1 and 10%. The weight ratio of *Bupleurum falcatum* to *Salvia milthiorriza* ranges between 1:200 and 10:1, and preferably between 1:10 and 10:1. Even more preferably, the ratio of *Bupleurum falcatum* to *Salvia milthiorriza* extracts is 1:2.

Preferably, *Bupleurumfalcatum* extract with a 1.5% content of total saikosaponins and *Salvia milthiorriza* lyophilized extract with an 0.09% content of diterpene quinones are used. For reasons of solubilization in the finished product, the latter extract can be replaced by a glycolic extract of *Salvia milthiorriza* with an 0.06% content of diterpene quinones, in an amount preferably between 1 and 40% by weight, and more preferably between 5 and 35%.

The compositions according to the invention can be prepared with conventional techniques and excipients and/or carriers, such as stabilizers, antioxidants, antibacterial and antifungal preservatives, colorants, perfumes and any other ingredient needed to give the product a particular physical form. The cosmetic compositions may take various physical forms, such as aerosols, sprays, fluid lotions, aqueous or hydroalcoholic gels, anhydrous gelified ointments, anhydrous oils, surfactant-containing systems, fluid or dense O/W and W/O emulsions and W/O/W or O/W/O triple emulsions, mud packs and sticks. They may also contain other active ingredients or substances, preferably of plant origin, designed to tone, firm, bleach, moisturise, and reduce stretch marks and roughness of the skin, such as centella, guarana, horse chestnut extracts and caffeine.

In particular, the carriers can be constituted by water-soluble substances and solvents such as water, lower alcohols, glycols and polyglycols, more or less gelified with plant polymers, synthetic polymers or mineral substances, either alone or in combination, or by lipbphilic substances such as vegetable oils, mineral oils, synthetic hydrocarbons, esters, alcohols and synthetic ethers. In particular, the following substances can be used: mono- and polyhydroxylic alcohols, esters of mono- and polyhydroxylic alcohols with acids having 2 to 26 carbon atoms, ethers of mono- and polyhydroxylic alcohols and mixtures thereof, saturated fatty acids totally or partly salified with strong or medium alkalis such as sodium and potassium hydroxide or triethanolamine, water- or alcohol-soluble polymers such as the Carbomers®, cellulose, their carboxymethyl and/or hydroxyalkyl derivatives, natural and modified starches, cyclodextrins, synthetic fluid, pasty or solid esters, such as isopropyl myristate and higher homologues, vegetable oils, volatile and non-volatile silicones, non-ionic, cationic and anionic surfactants and emulsifiers, either alone or mixed with the above-mentioned compounds to obtain solutions, emulsions or suspensions. Talc, sodium bicarbonate and kaolin, in the loose or compacted state, can also; be used as carriers,

The extracts in particular can be encapsulated or complexed to guarantee a controlled release and/or action, for example in beta-cyclodextrins, liposomes, micro sponges, microcapsules etc..

The preferred carrier for dissolution of the extracts is ethyl alcohol; if the composition takes the form of an aerosol foam, it will contain emulsifiers and/or surfactants in addition to ethyl alcohol.

In the case of compositions in the form of hydrophilic gels, ethyl alcohol (or other hydrophilic solvents) and water are used as the sole carriers of the extracts, the pH being buffered to a value suitable to stabilize the formulation.

In the case of compositions in the form of emulsions or microemulsions, oily suspensions or solutions of extracts in fluid oils for aerosols, it is preferable to use hydrophilic polymers which also have an emulsifying effect. These formulations can also contain phospholipids, so that liposomes containing the extracts are formed in both the inner and the outer phase. In this form, the active substances contained in the extracts can be released slowly, ensuring a constant, long-lasting effect. The slow-release effect can also be obtained with the use of microsponges.

Compositions with a solid consistency can be obtained by using, for example, lipid substances and silicones, in the presence of suitable mineral gelling agents and ethoxylated derivatives, In the case of pastes, extracts in non-alcoholic carriers in the presence of organic gelling agents will preferably be used.

Plant powders can be advantageously formulated in the form of mud packs with suitable excipients.

The compositions according to the invention can be packaged in containers at normal pressure, sprays or aerosols, tubes or jars, made of different materials.

Solutions, suspensions or emulsions can also be pressurised in aerosol canisters with one or more compartments to facilitate delivery in the form of a fluid, micronised droplets, paste or quick-breaking foam.

It has been observed that the cosmetic compositions and combinations according to this invention reduce blemishes caused by cellulite and the associated skin parameters, or at least limit their extent, to a far greater extent than that resulting from separate administration of each plant component.

In particular, the combinations according to the invention offer the following advantages:
- anti-oedema action, improved circulation at the skin surface, improved capillary structure of the superficial microcirculation, reduced amount of liquids in the spaces between the cells, and reduced skin tension, with an increase in elasticity parameters;
- an improvement in the outer appearance of the skin surface and treatment of the visible surface symptoms of cellulite (blemishes, dimpled, rippled appearance, orange-peel skin, and thigh circumference).

The invention will be now illustrated by the following examples.

### EXAMPLE

### Characterization methods

The roots used (Bupleurum spp and *Salvia milthiorriza*) were identified and characterized by means of phaimacognosy assays and titration of the main active principles; in particular saikosaponins A, C and D of *Bupleurum* spp as well as diterpenequinones of *Salvia milthiorriza* (cryptotanshinone, tanshinone I, tanshinone IIA) were titrated by HPLC. The same kind of titrations was also used for the characterization and standardization of the resulting extracts.

### Example 1: Slow-evaporation hydro-alcoholic gel

| | |
|---|---|
| *Bupleurum falcatum* lyophilized extract | 0.1 |
| *Salvia milthiorriza* lyophilized extract | 0-2 |
| Ethanol | 40 |
| Hydroxyethyl cellulose | 0.5 |
| Buffer solution to pH 5.5 | 40 |
| Additives (perfum, dye) | q.s. |
| Demineralized water | q.s. to 100 |

### Example 2: Hydro-alcoholic foam

| | |
|---|---|
| *Bupleurum falcatum* alcoholic extract | 0.05 |
| *Salvia milthiorriza* lyophilized extract | 0.3 |
| Ethanol | 30 |
| Hydroxyethyl cellulose | 0.5 |
| Emulsifier/surfactant | 2 |
| Buffer solution to pH 6 | 30 |
| Additives (perfum, dye) | q.s. |
| Demineralized water | q.s. to 100 |

The foam is subsequently distributed in aerosol containers with propane-butane propeller and propeller: base 5: 95 filling ratio.

### Example 3: Lotion

| | |
|---|---|
| *Bupleurum falcatum* glycolic extract | 0.1 |
| *Salvia milthiorriza* glycolic extract | 0.2 |
| Dimethyl-isosorbide | 30 |
| Carbomer® 941 | 0.5 |
| Emulsifier/surfactant additive | 2 |
| Buffer solution to pH 6 | 30 |
| Additives (perfum, dye, preservatives) | q.s. |
| Demineralized water | q.s. to 100 |

### Example 4: Lotion

| | |
|---|---|
| *Bupleurum* spp extract in almond oil | 0.1 |
| *Salvia milthiorriza* glycolic extract | 20 |
| Buffer solution to pH 5.5 (buffer citric) | 40 |
| Ethyl alcohol (95%) | q.s. to 100 |

| | |
|---|---|
| *Bupleurum falcatum* titrated lyophilized extract | 0.5 |
| *Salvia milthiorriza* glycolic extract | 10 |
| Ethyl alcohol (95%) | 40 |
| Carbomer® 940 | 1 |
| Ethoxylated oleyl alcohol | 3 |
| Buffer solution to pH 6 | 30 |
| Additives (perfum, dye, preservatives) | q.s. |
| Demineralized water | q.s. to 100 |

### Example 5: Hydro-Alcoholic emulsion

| | |
|---|---|
| *Bupleurum falcatum* titrated lyophilized extract | 0.5 |
| *Salvia milthiorriza* glycolic extract | 10 |
| Ethyl alcohol (95%) | 40 |
| Carbomer® 940 | 1 |
| Ethoxylated oleyl alcohol | 3 |
| Buffer solution to pH 6 | 30 |
| Additives (perfum, dye, preservatives) | q.s. |
| Demineralized water | q.s. to 100 |

### Example 6: Oily suspension

| | |
|---|---|
| *Bupleurum falcatum* oily extract in IPM | 0.4 |
| *Salvia milthiorriza* lyophilized extract | 0.3 |
| Additives (perfum, antioxidants) | q.s. |
| Isopropyl myristate | q.s. to 100 |

The mixture was pressurized , with propan-butane in a propeller: base 40: 60 mixing ratio.

### Example 7: Dispersion

| | |
|---|---|
| *Bupleurum falcatum* titrated lyophilized extract | 0.2 |
| *Salvia milthiorriza* lyophilized extract | 0.4 |
| Carbomer® 1342 | 1 |
| Ethoxylated sorbitan monolaurate | 3 |
| Buffer solution to pH 6 | 30 |
| Excipients (perfum, dye, preservatives) | q.s. |
| Demineralized water | q.s. to 100 |

### Example 8: Gel

| | |
|---|---|
| *Bupleurum falcatum* propylene glycol extract | 0.5 |
| *Salvia milthiorriza* lyophilized extract | 0.1 |
| Xanthan gum | 1 |
| Hydrogenated soy phospholipids | 5 |
| Buffer solution to pH 6 | 30 |
| Additives (perfum, dye, preservatives) | q.s. |
| Demineralized water | q.s. to 100 |

### Example 9: Hydro-Alcoholic gel with micro-sponges

| | |
|---|---|
| *Bupleurum falcatum* extracted in hazel oil | 0.1 |
| *Salvia milthiorriza* glycolic extract | 5 |
| Ethanol | 40 |
| Hydroxypropyl cellulose | 0.5 |
| Polythene microsponges | 2 |
| Buffer solution to pH 6 | 30 |
| Additives (perfum, dye, preservatives) | q.s. |
| Demineralized water | q.s. to 100 |

### Example 10: Hydro-Alcoholic gel

| | |
|---|---|
| *Bupleurum spp* alcoholic extract | 0.1 |
| *Salvia milthiorriza* lyophilized extract | 0.2 |
| Ethanol | 50 |
| Hydroxyethyl cellulose | 0.4 |
| 1,3-Butanediol | 2 |
| Buffer solution to pH 6 | 30 |
| Ethoxylated hydrogenated castor oil | 2 |
| Additives (perfum, dye, preservatives) | q.s. |
| Demineralized water | q.s. to 100 |

### Example 11: Mud gel

| | |
|---|---|
| *Bupleurum spp* powder | 0.5 |
| *Salvia milthiorriza* lyophilized extract | 0.7 |
| Pyrogenic silica | 2 |
| Centella, Salvia, Guarana, horse chestnut extracts | 5 |
| Caffeine | 0.5 |
| Additives (perfum, preservatives) | q.s. |
| Isagel® FM | q.s. to 100 |

### Example 12: Hydro-alcoholic stick

| | |
|---|---|
| *Bupleurum falcatum* alcoholic extract | 0.1 |
| *Salvia milthiorriza* lyophilized extract | 0.2 |
| Propylene glycol | 20 |
| Ethanol | 10 |
| Sodium stearate | 7 |
| Cetyl alcohol | 10 |
| Additives (perfum, preservatives) | q.s. |
| PPG-14-butyl ether | q.s. to 100 |

### Example 13: Non-alcoholic stick

| | |
|---|---|
| *Bupleurum falcatum* extract in oleyl alcohol | 0.15 |
| *Salvia milthiorriza* glycolic extract | 0.2 |
| PEG 200 | 20 |
| Propylene glycol | 10 |
| Laureth 3 | 10 |
| 2-Octyl dodecanol | 5 |
| Cetyl-stearyl alcohol | 1 |
| Cyclomethicone and Al, Mg idrossistearato | 20 |
| Cyclomethicone | 20 |
| Additives (perfum, preservatives) | q.s. |
| Dipropylene glycol | q.s. to 100 |

### Example 14: Cream

| | |
|---|---|
| *Bupleurum falcatum* lyophilized extract i | 0.8 |
| *Salvia milthiorriza* lyophilized extract | 0.8 |
| Gliceryl stearate | 4 |
| Isopropyl palmitate | 4 |
| Cetyl alcohol | 2 |
| Ethoxylated cetyl alcohol 20 OE | 2 |
| Bees wax | 1 |
| Buffer to pH 5.5 | 30 |
| Additives (perfum, dye, preservatives) | q.s. |
| Water | q.s. to 100 |

### Example 15: Powder for reconstitution with water

| | |
|---|---|
| *Bupleurum falcatum* polvere | 0.5 |
| *Salvia milthiorriza* lyophilized extract | 1.0 |
| Rice starch | 20 |
| Zinc oxide | 5 |
| Pyrogenic silica | 4 |
| Additives (perfum, dye, presevaltives) | q.s. |
| Talc | q.s. to 100 |

### Example 16: Roll-on formulation

| | |
|---|---|
| *Bupleurum falcatum* titrated lyophilized extract | 0.1 |
| *Salvia milthiorriza* lyophilized extract | 0.2 |
| Acrylate/C₁₀-C₃₀ alkyl acrylate cross-linked polymer | 0.5 |
| Hydroxyethyl cellulose | 0.5 |
| Ethoxylated hydrogenated castor oil 40 OE | 4 |
| Buffer solution to pH 5.5 | 30 |
| Additives (perfum, dye, preservatives) | q.s. |
| Water | q.s. to 100 |

### Example 17: Leg formulation

| | |
|---|---|
| *Bupleurum falcatum* titrated lyophilized extract | 0.15 |
| *Salvia milthiorriza* lyophilized extract | 0.25 |
| Propylene glycol | 5 |
| Ethoxylated cetylstearyl alcohol | 3 |
| Meadowfoam oil (*Limnanthes alba* oil) | 10 |
| Buffer solution to pH 5.5 | 30 |
| Menthyl lactate | 1 |
| Additives (perfum, dye, preservatives) | q.s. |
| Water | q.s. to 100 |

### Example 18: Detergent

| | |
|---|---|
| *Bupleurum falcatum* titrated lyophilized extract | 0.1 |
| *Salvia milthiorriza* glycolic extract | 10 |
| Anionic surfactants | 25 |
| Non-ionic surfactants | 15 |
| Cationic surfactants | 2 |
| Excipients (perfum, dyes, preservatives, buffers) | q.s. |
| Demineralized water | q.s. to 100 |

### Example 19: Water-swellable mixture

| | |
|---|---|
| *Bupleurum* spp powder | 2.0 |
| *Salvia milthiorriza* glycolic extract | 8.0 |
| Non-ionic surfactants | 10 |
| Green clay | 50 |
| Additives (perfum, dyes, preservatives, buffers) | q.s. |
| Magnesium aluminum silicate | q.s. to 100 |

### EVALUATION OF EFFICACY

### Activity test

Efficacy evaluations express the differences between the starting and final stage of the measurements in dimensional terms and in terms of the skin parameters associated with cellulite. For example, the efficacy of the composition referred to in example 15 was evaluated with a clinical test on 20 volunteers. This composition Was selected because it contained a lower level of *Bupleurum falcatum* titrated lyophilized extract and *Salvia milthiorriza* lyophilized extract than all the other examples reported. This composition was compared with an identical solution wherein the mixture of extracts was replaced with water.

The tests were performed alter 45 and 60 days' use of the products on 10 volunteers (mean age: 39 years). The following measurements were taken:
- measurement of thigh circumference;
- improvement in cutaneous microcirculation (Laser-doppler Flowmeter);
- elasticity (Courage and Kazaka elastometer);
- image analysis (skin tests) with resin from casts and computerised analysis of casts obtained for macro-roughness and micro-roughness parameters).

### RESULTS

### Measurement of thigh circumference

mean reduction after 30 days: 1.2 cm;
mean reduction after 45 days: 2.2 cm.

### Improvement in cutaneous microcirculation (the measurements were taken 30 min. after the last application)

mean increase after 30 days: + 12%;
mean increase after 45 days; 18%.

### Elasticity

mean elasticity: + 13.5% after 30 days; + 24.0% alter 45 days;
mean viscoelasticity: + 14.9% after 30 days, + 21.2% after 45 days.

### Image analysis

average reduction in macro-roughness after 30 days: +7%;
average reduction in macro-roughness after 45 days: + 11%;
increase in micro-roughness after 30 days: + 5%, after 45 days + 8%.

## Claims

1. The use of combinations of *Bupleurum* species extracts and *Salvia milthiorriza* extracts for the preparation of cosmetic compositions for the treatment of cellulite.

2. Cosmetic compositions for the treatment of cellulite containing combinations of *Bupleurum* species extracts and *Salvia milthiorriza* extracts in combination with suitable excipients and/or carriers.

3. Compositions as claimed in claim 2 **characterized in that** the total content in *Bupleurum falcatum* and *Salvia milthiorriza* extracts ranges from 0.1% to 30% by weight.

4. Compositions as claimed in claim 3 **characterized in that** the total content in *Bupleurum falcatum* and *Salvia milthiorriza* extracts ranges from 0.1 to 10% by weight.

5. Compositions according; to any one of claims 1-4 in which the *Bupleurum falcatum* to *Salvia milthiorriza* extracts weight ratio ranges from 1:200 to 10:1.

6. Compositions as claimed!in claim 5 in which the *Bupleurum falcatum* to *Salvia milthiorriza* extracts weight ratio ranges from 1:10 to 10:1.

7. Compositions as claimed in claim 6 in which the *Bupleurum falcatum* to *Salvia milthiorriza* extracts weight ratio is 1:2,

8. Compositions according to any one of claims 1-7 further containing one or more of the following substances: Centella, Guarana, horse chestnut extracts and caffeine.

9. Compositions according to any one of claims 1-8 in the form of aerosols, sprays, fluid lotions aqueous or hydroalcoholic gels, anhydrous gelified ointments, anhydrous oils, surfactant systems, fluid or dense O/W and W/O emulsions and W/O/W or O/W/O triple emulsions, mud packs and sticks.

10. A method for the cosmetic treatment of cellulite blemishes comprising the use of the compositions of any one of claims 1-9.
